# EUROPEAN PATENT SPECIFICATION

(11) **EP 3 969 000 B1**
(45) Date of publication and mention of the grant of the patent: **30.08.2023**
(21) Application number: 20723447.7
(22) Date of filing: 11.05.2020
(51) Int. Cl.: A61K 31/635, A61K 31/5377, A61K 45/06, A61P 35/00, A61P 35/02

(54) **BCL-2 INHIBITORS FOR USE IN THE TREATMENT OF A BCL-2 MEDIATED CANCER CARRYING THE GLY101VAL MUTATION**
BCL-2-INHIBITOREN ZUR VERWENDUNG BEI DER BEHANDLUNG VON BCL-2-VERMITTELTEM, DIE GLY101VAL-MUTATION TRAGENDEM KREBS
INHIBITEURS DE BCL-2 POUR UNE UTILISATION DANS LE TRAITEMENT D'UN CANCER PAR MÉDIATION BCL-2 PORTANT LA MUTATION GLY101VAL

(30) Priority: 14.05.2019 US 201962847477 P; 07.06.2019 EP 19178908; 07.02.2020 US 202062971297 P
(43) Date of publication of application: 23.03.2022
(73) Proprietor: Les Laboratoires Servier, 92284 Suresnes (FR); Novartis AG, 4056 Basel (CH)
(72) Inventor: MURRAY, James, Cambridgeshire CB21 4YL (GB); COLLAND, Frédéric, 95380 Puiseux-en-France (FR); CLAPERON, Audrey, 92400 Courbevoie (FR)
(86) International application number: PCT/EP2020/063089
(87) International publication number: WO 2020/229429

(56) References cited:
- US-A1- 2015 031 673
- Patrick Casara ET AL: "S55746 is a novel orally active BCL-2 selective and potent inhibitor that impairs hematological tumor growth", Oncotarget, 13 April 2018 (2018-04-13), pages 20075-20088, XP055643389, United States DOI: 10.18632/oncotarget.24744 Retrieved from the Internet: URL:http://www.oncotarget.com/index.php?jo urnal=oncotarget&page=article&op=view&path []=24744&pubmed-linkout=1 [retrieved on 2019-11-18]
- PIERS BLOMBERY ET AL: "Acquisition of the Recurrent Gly101Val Mutation in BCL2 Confers Resistance to Venetoclax in Patients with Progressive Chronic Lymphocytic Leukemia", CANCER DISCOVERY, vol. 9, no. 3, 1 March 2019 (2019-03-01), pages 342-353, XP55711262, US ISSN: 2159-8274, DOI: 10.1158/2159-8290.CD-18-1119
- RICHARD W. BIRKINSHAW ET AL: "Structures of BCL-2 in complex with venetoclax reveal the molecular basis of resistance mutations", NATURE COMMUNICATIONS, vol. 10, no. 1, 3 June 2019 (2019-06-03), XP055643364, DOI: 10.1038/s41467-019-10363-1

## Description

### FIELD OF THE INVENTION

The invention relates to a Bcl-2 inhibitor for use in the treatment of a Bcl-2 mediated cancer carrying at least 1, 2, 3, 4, 5 or all of the following mutations: (i) Gly101Val; (ii) Asp103Tyr; (iii) Asp103Val; (iv) Asp103Glu; (v) Arg129Leu and (vi) Ala113Gly; wherein the Bcl-2 inhibitor is *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide (Compound A, also known as S55746 or BCL201) or 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-N-(4-hydroxyphenyl)-1,2-dimethyl-1*H*-pyrrole-3-carboxamide (Compound B), or a pharmaceutically acceptable salt thereof. The invention also relates to pharmaceutical composition comprising Compound A or Compound B for use in the treatment of Bcl-2 mediated cancer carrying at least 1, 2, 3, 4, 5 or all of the following mutations: (i) Gly101Val; (ii) Asp103Tyr; (iii) Asp103Val; (iv) Asp103Glu; (v) Arg129Leu and (vi) Ala113Gly. In a further embodiment, the Bcl-2 mediated cancer carrying at least 1, 2, 3, 4, 5 or all of the following mutations: (i) Gly101 Val; (ii) Asp103Tyr; (iii) Asp103Val; (iv) Asp103Glu; (v) Arg129Leu and (vi) Ala113Gly is Chronic Lymphocytic Leukemia (CLL).

### BACKGROUND OF THE INVENTION

Apoptosis, or programmed cell death, is a physiological process that is crucial for embryonic development and maintenance of tissue homeostasis. Apoptotic-type cell death involves morphological changes such as condensation of the nucleus, DNA fragmentation and also biochemical phenomena such as the activation of caspases which cause damage to key structural components of the cell, so inducing its disassembly and death. Regulation of the process of apoptosis is complex and involves the activation or repression of several intracellular signalling pathways (Cory S. et al., Nature Review Cancer, 2002, 2, 647-656).

Deregulation of apoptosis is involved in certain pathologies. Increased apoptosis is associated with neurodegenerative diseases such as Parkinson's disease, Alzheimer's disease and ischaemia. Conversely, deficits in the implementation of apoptosis play a significant role in the development of cancers and their chemoresistance, in auto-immune diseases, inflammatory diseases and viral infections. Accordingly, absence of apoptosis is one of the phenotypic signatures of cancer (Hanahan D. et al., Cell 2000, 100, 57-70). The anti-apoptotic proteins of the Bcl-2 family are associated with numerous pathologies. The involvement of proteins of the Bcl-2 family is described in numerous types of cancer, such as colon cancer, breast cancer, small-cell lung cancer, non-small-cell lung cancer, bladder cancer, ovarian cancer, prostate cancer, chronic lymphoid leukaemia, follicular lymphoma, myeloma, and prostate cancer. Overexpression of the anti-apoptotic proteins of the Bcl-2 family is involved in tumorigenesis, in resistance to chemotherapy and in the clinical prognosis of patients affected by cancer. There is, therefore, a therapeutic need for compounds that inhibit the anti-apoptotic activity of the proteins of the Bcl-2 family. Venetoclax (also known as ABT-199) is a selective Bcl-2 inhibitor that counteracts the interaction of Bcl-2 with BH3-only proteins thus inducing apoptosis. Venetoclax is approved (i) to treat adults with chronic lymphocytic leukemia (CLL) or small lymphocytic lymphoma (SLL), with or without 17p deletion, who have received at least one prior treatment and (ii) in combination with azacitidine, or decitabine, or low-dose cytarabine to treat adults with newly-diagnosed acute myeloid leukemia (AML) who are 75 years of age or older, or have other medical conditions that prevent the use of standard chemotherapy. However, a significant number of relapses are observed in CLL suggesting an acquired mechanism of resistance. In other high-affinity targeted therapies, specific mutations of the target have been demonstrated as responsible for resistance (Garraway & Janne, Cancer Discovery 2012, 2, 214-226). In different venetoclax-resistant derived leukemia and lymphoma cell lines, Bcl-2 mutations affecting its hydrophobic groove have been identified (Fresquet et al., Blood 2014, 123, 4111-4119; Tahir et al., BMC Cancer, 17:399). One mutation on Bcl-2 (Glycine substituted by a Valine in position 101: Gly101Val) was recently shown to be clinically relevant since identified in CLL samples from patients resistant to venetoclax. This Gly101Val mutation was associated with reduced venetoclax binding to the hydrophobic groove of Bcl-2 and resistance to venetoclax (Blombery et al., Cancer Discovery 2019, 9, 342-353). The Gly101Val mutation may also provide a potential biomarker for impeding clinical relapse. Based on these clinical data, there is a need to identify new therapeutic agents that can be used to treat cancer patients who carry the Gly101Val mutation, and especially refractory and relapsed patients. More recently, other mutations in Bcl-2 such as Asp103Tyr (Aspartic acid substituted by a Tyrosine in position 103, also called D103Y), Asp103Val (Aspartic acid substituted by a Valine in position 103, also called D103V), Asp103Glu (Aspartic acid substituted by a Glutamic acid in position 103, also called D103E), Ala113Gly (Alanine substituted by a Glycine in position 113, also called A113G), Arg129Leu (Arginine substituted by a Leucine in position 129, also called R129L) and Val156Asp (Valine substituted by a Aspartic acid in position 156, also called V156D) have been identified in CLL patients treated with venetoclax while absent from naive CLL patients (Tausch et al., Hematologica 2019, 9, e434-e437; Blombery et al., Blood 2020, 135(10), 773-777). Of note, genomic analysis demonstrates that one CLL patient could bear different Bcl-2 mutations (i.e. including G101V and D103Y). All mutations were observed to be present in different reads in NGS (Next Generation Sequencing) data, consistent with their presence in different leukemic cells (assuming heterozygosity) and with mutual exclusivity of the mutations (Blombery et al., Blood 2020, 135(10), 773-777).

There is a second generation of Bcl-2-specific inhibitors, including Compound A and Compound B, which have a partially overlapping but distinct Bcl-2 hydrophobic groove binding mode compared to venetoclax.

The chemical structure of Compound A (also known as S55746 or BCL201) is:

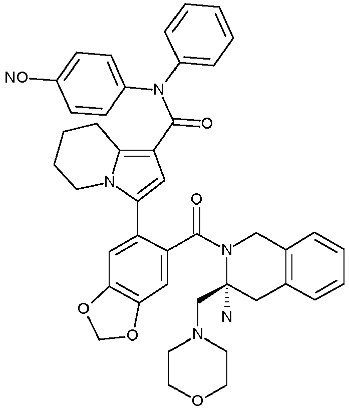

The preparation of Compound A and its pharmacological effects in several cancer models are described in the literature (Casara et al., Oncotarget 2018, Vol.9, No.28, 20075-20088 and corresponding Supplementary Information). Moreover, Compound A and structurallyclose analogues, their use as a Bcl-2 inhibitor for the treatment of cancer and pharmaceutical formulations thereof are also described in WO 2013/110890. The preparation of Compound A is specifically disclosed in Example 1 of that document in the form of a hydrochloride salt.

Compound A occupies the region typically referred to as S1/2/3 in contrast to the venetoclax analogue (Souers et al., Nature Medicine 2013, 19, 202-208), which occupies a greater portion of the protein surface area including S2/3/4/5. Compound A forms a single hydrogen bond to the backbone carbonyl of residue A149 buried deep into S2. The sizeindependent enthalpic efficiency (0.83) for Compound A binding to Bcl-2 is suggestive of optimal polar and Van der Waal's interactions, indicative of highly specific binding (Casara et al., Oncotarget 2018, 9, 20075-20088).

The structure of Compound B is: 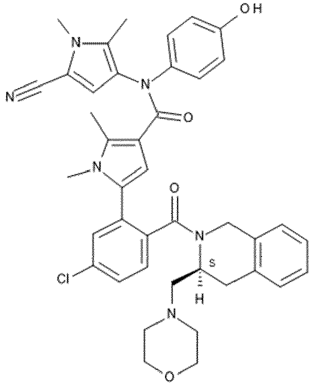 5-(5-chloro-2-{[(3*S*)-3-(morpholin-4-ylmethyl)-3,4-dihydroisoquinolin-2(1*H*)-yl]carbonyl} phenyl)-*N-*(5-cyano-1,2-dimethyl-1*H*-pyrrol-3-yl)-*N-*(4-hydroxyphenyl)-1,2-dimethyl-1*H-*pyrrole-3 -carboxamide.

The preparation of Compound B, its use as a Bcl-2 inhibitor for the treatment of cancer and pharmaceutical formulations thereof, are described in WO 2015/011400. Compound B is specifically disclosed in Example 386 of WO 2015/011400 in the form of a hydrochloride salt. It displays all the hallmarks of a Bcl-2 specific BH3-mimetic and exhibits robust antitumor activity in Bcl-2 dependent lymphoid tumor xenograft models while sparing platelets.

One assumption is that venetoclax on the one hand, and Compound A or Compound B on the other hand exhibit different binding modes. This structural hypothesis was confirmed by determining the different binding parameters of the three compounds in both wild-type Bcl-2 protein, Gly101Val, Asp103Tyr, Asp103Val, Asp103Glu, Arg129Leu and Ala113Gly mutant Bcl-2 proteins. The mutations in the Bcl-2 protein could thus be responsible for resistance to venetoclax but not to Compound A or Compound B. More particularly, Gly101Val mutation was located apart from the binding site of both Compound A and Compound B, in contrast to venetoclax. Furthermore, the Asp103Tyr mutation removes a key hydrogen bond between Bcl-2 and ABT-199 substantially reducing the affinity of ABT-199 for Bcl-2 harbouring the Asp103Tyr mutation. Compound B does not make any direct interaction with D103, it binds at least 9 angstroms away from D103, and thus its affinity for Bcl-2 harbouring the Asp103Tyr mutation is only modestly affected.

The Bcl-2 inhibitors have pro-apoptotic properties making it possible to use them in pathologies involving a defect in apoptosis, i.e. in the treatment of cancer and of immune and auto-immune diseases, and more specifically in patients resistant to venetoclax due to Bcl-2 mutations affecting its hydrophobic groove.

### SUMMARY

The invention is defined by the appended claims. Any subject-matter falling outside the scope of the claims is provided for information purposes, only.

The references to methods of treatment in this description are to be interpreted as references to the compounds, pharmaceutical compositions and medicaments of the present invention for use in a method for treatment of the human (or animal) body by therapy (or for diagnosis).

The present invention provides a Bcl-2 inhibitor for use in the treatment of Bcl-2 mediated cancer carrying at least 1, 2, 3, 4, 5 or all of the following mutations: (i) Gly101Val; (ii) Asp103Tyr; (iii) Asp103Val; (iv) Asp103Glu; (v) Arg129Leu and (vi) Ala113Gly; wherein the Bcl-2 inhibitor is Compound A, Compound B, or a pharmaceutically acceptable salt thereof. In particular, it has been found that the Bcl-2 inhibitors according to the invention have a strong activity for Bcl-2 Gly101Val mutant. The slight loss of activity observed between the wild type Bcl-2 protein and the Gly101Val mutant suggests that the administration of the Bcl-2 inhibitor according to the invention could induce a clinically relevant response in patients carrying the Gly101Val mutation. Additional cellular studies further confirmed that Compound B only showed a slight loss of potency (9 to 7 fold) in cell lines harboring the Bcl-2 Gly101Val mutant as compared to cell lines expressing the Bcl-2 wild-type protein. More generally, considering the biochemical profile disclosed herein, Compound B was found to display a better on-target activity on the set of mutants discussed above as compared to ABT-199. In addition to the biochemical data, cellular studies further demonstrated a shift of potency of 22-fold between ABT-199 and Compound B in cellular assays using KMS-12-PE cell lines overexpressing Bcl-2 Asp103Tyr. The present disclosure as a whole suggests that the Bcl-2 inhibitors according to the invention - and more especially Compound B - may have a beneficial effect in patients harbouring at least 1, 2, 3, 4, 5 or all of the following mutations: (i) Gly101Val; (ii) Asp103Tyr; (iii) Asp103Val; (iv) Asp103Glu; (v) Arg129Leu and (vi) Ala113Gly. In a particular aspect, CLL patients who have acquired one of the previous mutations further to venetoclax treatment are targeted.

In another aspect, the invention relates to a method for sensitizing a patient having a Bcl-2 mediated cancer carrying at least 1, 2, 3, 4, 5 or all of the following mutations: (i) Gly101 Val; (ii) Asp103Tyr; (iii) Asp103Val; (iv) Asp103Glu; (v) Arg129Leu and (vi) Ala113Gly; who is (i) refractory to at least one anti-cancer agent, or (ii) in relapse after treatment with an anti-cancer agent, or both (i) and (ii), wherein the method comprises administering a therapeutically effective amount of a Bcl-2 inhibitor to said patient.

Overall, the invention described herein could enable to administrate a therapeutically effective amount of a composition including Compound A or Compound B to refractory or relapsed cancer patients, who carry at least 1, 2, 3, 4, 5 or all of the following mutations: (i) Gly101 Val; (ii) Asp103Tyr; (iii) Asp103Val; (iv) Asp103Glu; (v) Arg129Leu and (vi) Ala113Gly; including venetoclax resistant patients.

### DETAILED DESCRIPTION OF THE INVENTION

`Compound A' means *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide.

`Compound A, HCl' means *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N-*phenyl-5,6,7,8-tetrahydro-1-indolizine carboxamide in the form of a hydrochloride salt.

`Compound B' means 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide.

`Compound B, HCl' means that 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N-*(4-hydroxyphenyl)-1,2-dimethyl-1*H*-pyrrole-3-carboxamide is in the form of a hydrochloride salt.

`Compound B, H₂SO₄' means that 5-(5-chloro-2-{[(3S)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide is in the form of a hydrogen sulfate salt.

`Free molecule' and `free base' are used interchangeably herein and refer to Compound A or Compound B when not in salt form.

"Cancer" means a class of disease in which a group of cells display uncontrolled growth. Cancer types include haematological cancer (lymphoma and leukaemia) and solid tumors including carcinoma, sarcoma, or blastoma. In particular "cancer" refers to leukaemia, lymphoma or multiple myeloma, and more especially to chronic lymphocytic leukaemia, non Hodgkin lymphoma (including follicular lymphoma) or acute myeloid leukaemia.

`Bcl-2 mediated cancer' means a cancer in which the Bcl-2 protein can act as barrier to apopotosis and facilitate tumour development and resistance to cancer therapy. In particular, `Bcl-2 mediated cancer' includes cancer characterized by a dysregulation of the Bcl-2 protein expression.

`HP- β-cyclodextrin' is also named `hydroxypropyl- β-cyclodextrin' or `2-hydroxypropyl-β-cyclodextrin' or `hydroxypropylbetadex'. In particular, the HP- β-cyclodextrin is marketed with the following product names: Cavitron^{™} W7HP7 (typical degree of substitution: 6.0-8.0 ; approximate molecular weight: 1520), Cavitron^{™} W7HP5 (typical degree of substitution: 4.1-5.1 ; approximate molecular weight: 1410), Kleptose^{™} HPB or Kleptose^{™} HP.

As used herein, the term 'comprising' means 'including', and is not intended to exclude the presence of any additional component, unless the context suggests otherwise, for example when the components together sum to 100 %.

As used herein, the term 'treat', 'treating' or 'treatment' of any disease or disorder refers in one embodiment, to ameliorating the disease or disorder (*i.e.*, slowing or arresting or reducing the development of the disease or at least one of the clinical symptoms thereof). In another embodiment, 'treat', 'treating' or 'treatment' refers to alleviating or ameliorating at least one physical parameter including those which may not be discernible by the patient. In yet another embodiment, 'treat', 'treating' or 'treatment' refers to modulating the disease or disorder, either physically, (*e.g*., stabilization of a discernible symptom), physiologically, (*e.g*., stabilization of a physical parameter), or both.

As used therein, a "therapeutically effective amount of the composition" means an effective amount of the composition according to the invention containing an effective dose of active principle to elicit a therapeutic benefit for the patient. For Compound A, the useful dosage ranges from 50 mg to 1500 mg per day expressed in terms of the free base. The dose of Compound B administered according to the invention is from 5 mg to 1000 mg expressed as free base.

As used therein, "a method for sensitizing" means a therapeutic method that allows ameliorating the disease or disorder (*i.e*., slowing or arresting or reducing the development of the disease or at least one of the clinical symptoms thereof) in relapsed or refractory patients. In a particular embodiment, "a method for sensitizing" means the restoration of a clinical response in patients resistant to an existing therapy.

As used therein, "targeted therapy" means a therapy that blocks the growth of cancer cells by interfering with specific targeted molecules needed for carcinogenesis and tumor growth, rather than by simply interfering with all rapidly dividing cells (as with traditional chemotherapy).

Described below are a number of embodiments of the invention.

In one embodiment, the present invention relates to a Bcl-2 inhibitor and some pharmaceutical compositions containing it, for use in the treatment of Bcl-2 mediated cancer carrying at least 1, 2, 3, 4, 5 or all of the following mutations: (i) Gly101Val; (ii) Asp103Tyr; (iii) Asp103Val;(iv) Asp103Glu; (v) Arg129Leu and (vi) Ala113Gly; wherein the Bcl-2 inhibitor is selected from the group consisting of *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide (Compound A) and 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide (Compound B), or a pharmaceutically acceptable salt thereof.

In another embodiment, the present invention relates to a Bcl-2 inhibitor and some pharmaceutical compositions containing it, for use in the treatment of Bcl-2 mediated cancer wherein the cancer carries the Gly101Val mutation.

In another embodiment, the present invention relates to a Bcl-2 inhibitor and some pharmaceutical compositions containing it, for use in the treatment of Bcl-2 mediated cancer wherein the cancer carries the Asp103Tyr mutation.

In another embodiment, the present invention relates to a Bcl-2 inhibitor and some pharmaceutical compositions containing it, for use in the treatment of Bcl-2 mediated cancer wherein the cancer carries the Asp103Val mutation.

In another embodiment, the present invention relates to a Bcl-2 inhibitor and some pharmaceutical compositions containing it, for use in the treatment of Bcl-2 mediated cancer wherein the cancer carries the Asp103Glu mutation.

In another embodiment, the present invention relates to a Bcl-2 inhibitor and some pharmaceutical compositions containing it, for use in the treatment of Bcl-2 mediated cancer wherein the cancer carries the Arg129Leu mutation.

In another embodiment, the present invention relates to a Bcl-2 inhibitor and some pharmaceutical compositions containing it, for use in the treatment of Bcl-2 mediated cancer wherein the cancer carries the Ala113Gly mutation.

In a preferred embodiment, the Bcl-2 mediated cancer is chronic lymphocytic leukemia (CLL). Alternatively, the Bcl-2 mediated cancer is acute myeloid leukaemia (AML), multiple myeloma or non Hodgkin lymphoma.

In some embodiments, the Bcl-2 inhibitor is Compound A,which is in the form of a hydrochloride salt.

In some embodiments, the Bcl-2 inhibitor is Compound B, which is in the form of a hydrogen sulfate salt.

In other embodiments, the Bcl-2 inhibitor is Compound B, which is in the form of a hydrochloride salt.

In some embodiments, the Bcl-2 inhibitor is Compound B, which is administered intravenously.

In some embodiments, the invention relates to a method for sensitizing a patient having a Bcl-2 mediated cancer carrying at least 1, 2, 3, 4, 5 or all of the following mutations: (i) Gly101 Val; (ii) Asp103Tyr; (iii) Asp103Val; (iv) Asp103Glu; (v) Arg129Leu and (vi) Ala113Gly; who is (a) refractory to at least one anti-cancer agent, or (b) in relapse after treatment with an anti-cancer agent, or both (a) and (b), wherein the method comprises administering a therapeutically effective amount of the Bcl-2 inhibitor as defined previously, to said patient.

In some embodiments, the invention relates to a method for sensitizing a patient having a Bcl-2 mediated cancer carrying the Gly101Val mutation who is (i) refractory to at least one anti-cancer agent, or (ii) in relapse after treatment with an anti-cancer agent, or both (i) and (ii), wherein the method comprises administering a therapeutically effective amount of the Bcl-2 inhibitor as defined previously, to said patient.

In some embodiments, the invention relates to a method for sensitizing a patient having a Bcl-2 mediated cancer carrying the Asp103Tyr mutation who is (i) refractory to at least one anti-cancer agent, or (ii) in relapse after treatment with an anti-cancer agent, or both (i) and (ii), wherein the method comprises administering a therapeutically effective amount of a Bcl-2 inhibitor as defined previously, to said patient.

In some embodiments, the invention relates to a method for sensitizing a patient having a Bcl-2 mediated cancer carrying the Asp103Val mutation who is (i) refractory to at least one anti-cancer agent, or (ii) in relapse after treatment with an anti-cancer agent, or both (i) and (ii), wherein the method comprises administering a therapeutically effective amount of a Bcl-2 inhibitor as defined previously, to said patient.

In some embodiments, the invention relates to a method for sensitizing a patient having a Bcl-2 mediated cancer carrying the Asp103Glu mutation who is (i) refractory to at least one anti-cancer agent, or (ii) in relapse after treatment with an anti-cancer agent, or both (i) and (ii), wherein the method comprises administering a therapeutically effective amount of a Bcl-2 inhibitor as defined previously, to said patient.

In some embodiments, the invention relates to a method for sensitizing a patient having a Bcl-2 mediated cancer carrying the Arg129Leu mutation who is (i) refractory to at least one anti-cancer agent, or (ii) in relapse after treatment with an anti-cancer agent, or both (i) and (ii), wherein the method comprises administering a therapeutically effective amount of a Bcl-2 inhibitor as defined previously, to said patient.

In some embodiments, the invention relates to a method for sensitizing a patient having a Bcl-2 mediated cancer carrying the Ala113Gly mutation who is (i) refractory to at least one anti-cancer agent, or (ii) in relapse after treatment with an anti-cancer agent, or both (i) and (ii), wherein the method comprises administering a therapeutically effective amount of a Bcl-2 inhibitor as defined previously, to said patient.

In a preferred embodiment, the patient having the Bcl-2 mediated cancer is refractory to at least one targeted therapy, or (ii) in relapse after treatment with a targeted therapy, or both (i) and (ii). In a particular aspect, the Bcl-2 mediated cancer is chronic lymphocytic leukemia (CLL). In another aspect, the targeted therapy is venetoclax (ABT-199).

In some embodiments, the invention relates to a method of treating a Bcl-2 mediated cancer in a patient, comprising the steps of:
(a) obtaining a biological sample from said patient and detecting whether the biological sample comprises at least 1, 2, 3, 4, 5 or all of the following mutations: (i) Gly101Val; (ii) Asp103Tyr; (iii) Asp103Val; (iv) Asp103Glu; (v) Arg129Leu and (vi) Ala113Gly;
(b) identifying said patients as having reduced likelihood of response to venetoclax;
(c) administering a therapeutically effective amount of a Bcl-2 inhibitor as defined previously, to said patient based on the presence of the thus detected mutations.

In some embodiments, the invention relates to a method of treating a Bcl-2 mediated cancer in a patient, comprising the steps of:
(a) obtaining a biological sample from said patient and detecting whether the biological sample comprises the Gly101Val mutation;
(b) identifying said patients as having reduced likelihood of response to venetoclax;
(c) administering a therapeutically effective amount of a Bcl-2 inhibitor as defined previously, to said patient based on the presence of the Gly101Val mutation.

In some embodiments, the invention relates to a method of treating a Bcl-2 mediated cancer in a patient, comprising the steps of:
(a) obtaining a biological sample from said patient and detecting whether the biological sample comprises the Asp103Tyr mutation;
(b) identifying said patients as having reduced likelihood of response to venetoclax;
(c) administering a therapeutically effective amount of a Bcl-2 inhibitor as defined previously, to said patient based on the presence of the Asp103Tyr mutation.

In some embodiments, the invention relates to a method of treating a Bcl-2 mediated cancer in a patient, comprising the steps of:
(a) obtaining a biological sample from said patient and detecting whether the biological sample comprises the Asp103Val mutation;
(b) identifying said patients as having reduced likelihood of response to venetoclax;
(c) administering a therapeutically effective amount of a Bcl-2 inhibitor as defined previously, to said patient based on the presence of the Asp103Val mutation.

In some embodiments, the invention relates to a method of treating a Bcl-2 mediated cancer in a patient, comprising the steps of:
(a) obtaining a biological sample from said patient and detecting whether the biological sample comprises the Asp103Glu mutation;
(b) identifying said patients as having reduced likelihood of response to venetoclax;
(c) administering a therapeutically effective amount of a Bcl-2 inhibitor as defined previously, to said patient based on the presence of the Asp103Glu mutation.

In some embodiments, the invention relates to a method of treating a Bcl-2 mediated cancer in a patient, comprising the steps of:
(a) obtaining a biological sample from said patient and detecting whether the biological sample comprises the Arg129Leu mutation;
(b) identifying said patients as having reduced likelihood of response to venetoclax;
(c) administering a therapeutically effective amount of a Bcl-2 inhibitor as defined previously, to said patient based on the presence of the Arg129Leu mutation.

In some embodiments, the invention relates to a method of treating a Bcl-2 mediated cancer in a patient, comprising the steps of:
(a) obtaining a biological sample from said patient and detecting whether the biological sample comprises the Ala113Gly mutation;
(b) identifying said patients as having reduced likelihood of response to venetoclax;
(c) administering a therapeutically effective amount of a Bcl-2 inhibitor as defined previously, to said patient based on the presence of the Ala113Gly mutation.

### EXAMPLE 1: Affinity data of Compound A and Compound B on Bcl-2 wild-type and Bcl-2 Gly101Val mutant

Fluorescence quenching assay measures the change fluorescence intensity of:
(i) C-terminally Cy5-labelled Bcl-2 wild-type protein (UniProtKB^{®} primary accession number P10415) having an amino acid sequence (SEQ ID:02):
   [MGHHHHHHHHSAGLVPRGSMAHAGRTGYDNREIVMKYIHYKLSQRGY EWDAGDVGAAPPGAAPAPGIFSSQPGHTPHPAASRDPVARTSPLQTPAAP GAAAGPALSPVPPVVHLTLRQAGDDFSRRYRRDFAEMSSQLHLTPFTAR GRFATVVEELFRDGVNWGRIVAFFEFGGVMCVESVNREMSPLVDNIAL WMTEYLNRHLHTWIQDNGGWDAFVELY] which is linked at the C-terminus to the amino acid X which corresponds to a cysteine as defined below, or,
(ii) Bcl-2 Gly101Val mutant having an amino acid sequence (SEQ ID:03):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPPVVHLTLRQAVDDFSRRYRRDFAEMSSQLHLTPFT ARGRFATVVEELFRDGVNWGRIVAFFEFGGVMCVESVNREMSPLVDNI ALWMTEYLNRBLHTWIQDNGGWDAFVELY] which is linked at the C-terminus to the amino acid X which corresponds to a cysteine as defined below, (where X = is cysteine labelled on the sulfur with sulpho-Cyanine5 from Lumiprobe GmbH catalogue number 13380)
upon binding of a C-terminally labelled peptide derived from PUMA (UniProtKB^{®} primary accession number Q9BXH1) having an amino acid sequence (SEQ ID:01): [QWAREIGAQLRRMADDLNAQY] which is linked at the C-terminus to the amino acid X', where X' is cysteine labelled on the sulfur with TQSWS from AAT Bioquest catalogue number 2079.

The addition of a compound which binds competitively to the same site as the peptide will result in an increase in the fluorescence intensity of the protein due to displacement of the fluorescence quencher.

An 11-point serial dilution of each compound was prepared in DMSO, the final buffer conditions were 10 mM 4-(2-hydroxyethyl)-1-piperazineethanesulfonic acid [HEPES], 150 mM NaCl, 0.05% Tween 20, pH 7.4 and 5% DMSO. The final protein concentration in the assay was 1 nM with the peptide present at 50 nM (Bcl-2 wild-type) or 100 nM (Bcl-2 Gly101Val). The experiments were incubated for 2 hours at room temperature before fluorescence intensity was measured on a Biotek SynergyNeo plate reader (Excitation 620nm, emission 680nm). The dose response curves were plotted with XL-Fit software using a 4-Parameter Logistic Model (Sigmoidal DoseResponse Model) and the inhibitory concentrations that gave a 50% increase in fluorescence intensity was determined (IC₅₀). The K_{I} values were determined from the IC₅₀ values according to Cer et al, Nucleic Acids Res, 2009, Jul 1;37(WebServer issue): W441-W445.

| | Bcl-2 wild-type cKi mean (M) | Bcl-2 Gly101Val mutant cKi mean (M) | Ratio Mutant/Wild-type |
|---|---|---|---|
| ABT-199 | 1.1E-09 | 1.07E-07 | 97 |
| Compound A | 1.2E-09 | 2.37E-08 | 20 |
| Compound B | 1.0E-10 | 1.42E-09 | 14 |

The data demonstrate a significant loss of activity of ABT-199 on the Bcl-2 Gly101Val mutant as compared to the Bcl-2 wild-type protein, whilst the affinities of Compound A and Compound B are slightly affected by the mutation. Furthermore, Compound B is 75-fold more potent than ABT-199 on the Bcl-2 Gly101Val mutant.

### EXAMPLE 2: In vitro cytotoxicity of Compound A and Compound B in modified cells expressing either Bcl-2 wild-type or Bcl-2 Gly101Val mutant

### Material and methods

Cell lines were grown at 37 °C in a humidified atmosphere with 5% CO₂ in media recommended by the suppliers. RS4;11 (ATCC^{®} CRL1873^{™}) were purchased from American Type Culture Collection (ATCC) and KMS-12-PE (ACC 606) from the Leibniz-Institute DSMZ (Braunschweig, Germany). Lentiviral particles containing Bcl-2 wild-type (also named `Bcl-2 WT') and Bcl-2 mutated on G101V (also named `Bcl-2 G101V') were cloned into pcLV-CMV-DEST-IRES-TagRFP. Lentiviral particles (1 × 10⁶) were mixed with Polybrene at 8 µg/ml and transduced by spinoculation for 1h at 32 °C and incubated overnight. After 8 days for KMS-12-PE and 21 days for RS4;11, TagRFP positive-cells were sorted by FACS. BCL2 expression was monitored by immunoblotting using anti-Flag and anti-BCL2 antibodies. Cells were seeded into 96-well plates and treated with 1:3.16 serial dilution of compounds (9 different concentrations). Cell viability was assessed using CellTiter-Glo^{®} reagent following treatment with ABT-199, Compound A and Compound B for 72h. Results were normalized to the viability of cells without compounds (control wells). The C₅₀ values were calculated using nonlinear regression algorithms in XCell software.

### Results

| **Compounds** | **KMS-12-PE Bcl-2 WT (C50; nM)** | **KMS-12-PE Bcl-2 G101V (C50; nM)** | **Ratio (G101V/WT)** |
|---|---|---|---|
| Compound A | 57.3 | 961 | 17 |
| Compound B | 21 | 179 | 9 |
| ABT-199 | 22.5 | 931 | 41 |

| **Compounds** | **RS4;11 Bcl-2 WT (C50; nM)** | **RS4;11 Bcl-2 G101V (C50; nM)** | **Ratio (G101V/WT)** |
|---|---|---|---|
| Compound A | 82.3 | 495 | 6 |
| Compound B | 9.03 | 60.4 | 7 |
| ABT-199 | 10.7 | 130 | 12 |

### Conclusion

These results demonstrated a shift of potency between ABT-199 and Compound B in cellular assays using two different cell lines. Indeed, a significant loss of potency of ABT-199 was observed in cell lines overexpressing the Bcl-2 G101V mutant compared to the cell lines overexpressing the Bcl-2 wild-type protein (41 to 12-fold difference depending on cell lines). In contrast, Compound B showed only 9 to 7-fold difference between cell lines overexpressing either G101V Bcl-2 protein or wild type protein. While both Compound B and ABT-199 exhibited similar potency in both cell lines overexpressing wild type Bcl-2, Compound B showed 5 to 2-fold higher potency than ABT-199 in cell lines overexpressing the Bcl-2 G101V mutant.

### EXAMPLE 3: Clinical Trial Protocol

A phase I, open label, non-randomised, non-comparative, multi-center study, was set up to evaluate Compound B intravenously administered, in patients with Relapse or Refractory Acute Myeloid Leukaemia, Non Hodgkin Lymphoma, Multiple Myeloma or Chronic Lymphocytic Leukemia (CLL). In particular, the patients with CLL included in this study have relapsed or are refractory (except treatment failure, *e.g*. stable disease, non-response, progressive disease, death from any cause), as defined per iwCLL guidelines (Hallek M. et al, Blood, 2018, Vol. 131, 25, 2745-2760), from venetoclax treatment and without established alternative therapy. Approximately 60 patients will be enrolled in the study. This study is designed in two parts: part one for dose escalation, part two for dose expansion.

### Primary objectives:

Determine the safety profile (including Dose Limiting Toxicity (DLT) and Maximum Tolerated Dose (MTD(s)) and tolerability of Compound B in patients with Acute Myeloid Leukaemia (AML), Non Hodgkin Lymphoma (NHL), Multiple Myeloma (MM) or Chronic Lymphocytic Leukemia (CLL) and the recommended phase II dose (RP2D(s)) according to safety, PK and preliminary efficacy results.

### Secondary objectives:

- To determine the pharmacokinetic (PK) profile of Compound B in plasma and in urine.
- To assess the preliminary anti-tumor activity of Compound B using the appropriate response criteria for each evaluated population (AML, NHL, MM, CLL).

### Exploratory objectives relative to CLL patients with mutations:

- To assess the efficacy of Compound B on cells harboring Bcl-2 mutation(s), including the Gly101Val mutation and the Asp103Tyr mutation, by comparing pre- and ontreatment Bone Marrow Aspirate and blood samples in patients suffering from AML or CLL who previously received venetoclax. The mutations are detected by analyzing the patients samples using the Droplet Digital PCR technology (Vogelstein and Kinzler, Proc. Natl. Acad. Sci. USA 1999 96 Genetics; Olmedillas-López S et al, Mol Diagn Ther. 2017 Oct;21(5):493-510).

### Test drug:

- Compound B will be administered via i.v. infusion via a central or peripheral venous line.
- Solution for infusion will be prepared using a 20 mL vials containing 150 mg of Compound B (expressed as free base) formulated with a HP-β-cyclodextrin as described below.
- Duration of infusion, based on preliminary Safety and PK data, could be adapted.

### Preparation of lyophilisates of Compound B solubilised in a HP-β-cyclodextrin in 20 mL vials:

The lyophilisates are prepared in 20 mL vials in which it will be possible to reconstitute the solution to be administered by the parenteral route. They are obtained by lyophilisation of a 20% Cavitron^{™} W7HP5 solution containing a dose of 20 mg/mL of Compound B (free base).

### Procedure

In a 5 L reactor, weigh 1500 g of water. With magnetic stirring, create a vortex and then pour in 600 g of Cavitron^{™} W7HP5. Stir the medium at ambient temperature until the cyclodextrin is solubilised completely, and add 68.16 g of 'Compound B, H₂SO₄' and heat the solution to not more than 60 °C. Place the suspension under magnetic stirring for several hours and then allow the medium to return to a temperature below 30°C. Measure the pH of the solution so obtained, then adjust it to pH 3.0 with 0.5M NaOH solution poured slowly. Make up the solution to a volume of 3 L by adding water, while maintaining magnetic stirring.

Pass the solution so obtained through a 0.2 µm filter.

Fill the 20 mL vials with the filtered solution so that each vial contains at least 150 mg of Compound B (expressed as free base) and subject the samples to a lyophilisation step.

The resulting lyophilisate is intended to be used for the preparation of a pharmaceutical composition for parenteral administration.

### Dose allocation methodology:

A Bayesian Hierarchical Model (BHM), combined for all indications and guided by an escalation with overdose control (EWOC) method, will be used to guide dose escalation and estimate the MTD(s) based on the occurrence of DLT during Cycle 1.

Alternatively, an adaptative Bayesian Logistic Regression Model (BLRM) guided by an escalation with overdose control (EWOC) method, will be used to make dose recommendations based on the occurrence of DLT(s) during Cycle 1 and estimate the MTD(s)/RP2D(s) for the Compound B administered as a single agent.

### Treatment period:

The planned duration of treatment is until disease progression. Patients may be discontinued from treatment with the study drug earlier due to unacceptable toxicity and/or treatment is discontinued at the discretion of the investigator or the patient.

### EXAMPLE 4: Affinity data of Compound A and Compound B on Bcl-2 wild-type, Gly101Val mutant, Bcl-2 Asp103Tyr mutant. Asp103Val mutant. Asp103Glu mutant; Arg129Leu mutant and Ala113Gly mutant

Fluorescence quenching assay measures the change fluorescence intensity of:
(i) C-terminally Cy5-labelled Bcl-2 wild-type protein (UniProtKB^{®} primary accession number P10415) having an amino acid sequence (SEQ ID:02):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPPVVHLTLRQAGDDFSRRYRRDFAEMSSQLHLTPFT ARGRFATVVEELFRDGVNWGRIVAFFEFGGVMCVESVNREMSPLVDNI ALWMTEYLNRBLHTWIQDNGGWDAFVELY] which is linked at the C-terminus to the amino acid X which corresponds to a cysteine as defined below, or,
(ii) Bcl-2 Gly101Val mutant having an amino acid sequence (SEQ ID:03):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPPVVHLTLRQAVDDFSRRYRRDFAEMSSQLHLTPFT ARGRFATVVEELFRDGVNWGRIVAFFEFGGVMCVESVNREMSPLVDNI ALWMTEYLNRBLHTWIQDNGGWDAFVELY] which is linked at the C-terminus to the amino acid X which corresponds to a cysteine as defined below, or,
(iii) Bcl-2 Asp103Tyr mutant having an amino acid sequence (SEQ ID:04):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPP VVHLTLRQ AGDYF SRRYRRDF AEMS SQLHLTPFT ARGRFATVVEELFRDGVNWGRIVAFFEFGGVMCVESVNREMSPLVDNI ALWMTEYLNRBLHTWIQDNGGWDAFVELY] which is linked at the C-terminus to the amino acid X which corresponds to a cysteine as defined below, or,
(iv) Bcl-2 Asp103Val mutant having an amino acid sequence (SEQ ID:05):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPP VVHLTLRQ AGDVF SRRYRRDFAEMS SQLHLTPFT ARGRFATVVEELFRDGVNWGRIVAFFEFGGVMCVESVNREMSPLVDNI ALWMTEYLNRHLHTWIQDNGGWDAFVELY] which is linked at the C-terminus to the amino acid X which corresponds to a cysteine as defined below, or,
(v) Bcl-2 Asp103Glu mutant having an amino acid sequence (SEQ ID:06):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPP VVHLTLRQ AGDEF SRRYRRDFAEMS SQLHLTPFT ARGRFATVVEELFRDGVNWGRIVAFFEFGGVMCVESVNREMSPLVDNI ALWMTEYLNRHLHTWIQDNGGWDAFVELY] which is linked at the C-terminus to the amino acid X which corresponds to a cysteine as defined below, or,
(vi) Bcl-2 Arg129Leu mutant having an amino acid sequence (SEQ ID:07):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPP VVHLTLRQ AGDDF SRRYRRDFAEMS SQLHLTPFT ARGLFATVVEELFRDGVNWGRIVAFFEFGGVMCVESVNREMSPLVDNI ALWMTEYLNRBLHTWIQDNGGWDAFVELY] which is linked at the C-terminus to the amino acid X which corresponds to a cysteine as defined below, or,
(vii) Bcl-2 Ala113Gly mutant having an amino acid sequence (SEQ ID:08):
   [MGHHHHHHHHSAGLVPRGSMAHAGRTGYDNREIVMKYIHYKLSQRG YEWDAGDVGAAPPGAAPAPGIFSSQPGHTPHPAASRDPVARTSPLQTPA APGAAAGPALSPVPP VVHLTLRQ AGDDF SRRYRRDFGEMS SQLHLTPFT ARGRFATVVEELFRDGVNWGRIVAFFEFGGVMCVESVNREMSPLVDNI ALWMTEYLNRHLHTWIQDNGGWDAFVELY] which is linked at the C-terminus to the amino acid X, (where X = is cysteine labelled on the sulfur with sulpho-Cyanine5 from Lumiprobe GmbH catalogue number 13380)
upon binding of a C-terminally labelled peptide derived from PUMA (UniProtKB^{®} primary accession number Q9BXH1) having an amino acid sequence (SEQ ID:01): [QWAREIGAQLRRMADDLNAQY] which is linked in C-terminal region to the amino acid X', where X' is cysteine labelled on the sulfur with TQSWS from AAT Bioquest catalogue number 2079.

The addition of a compound which binds competitively to the same site as the peptide will result in an increase in the fluorescence intensity of the protein due to displacement of the fluorescence quencher.

The objective was to determine the K_{I} of ABT-199, Compound A and Compound B as competitive binders of recombinant Bcl-2 wild type, G101V, D103Y, D103V, D103E, A113G, R129L mutants via PUMA quench reagent displacement, measured by fluorescence intensity.

The assays were carried out in black-walled, flat bottomed, low binding, 384-well plates. Compound (final conc. 5% DMSO) was mixed in buffer (10 mM 4-(2-hydroxyethyl)-1-piperazineethanesulfonic acid [HEPES], 150 mM NaCl, 0.05% Tween 20, pH 7.4), containing 50 nM of the peptide (probe), except in the case of G101V where 100 nM of the peptide was used, and [1 nM Bcl-2 wild type protein or 1 nM Bcl-2 mutant]. Assay plates were incubated about 2 hours at 18°C and fluorescence intensity measured on a Synergy Neo reader (Ex. 620nm, Em. 680nm). The dose response curves were plotted with XL-Fit software using a 4-Parameter Logistic Model (Sigmoidal DoseResponse Model) and the inhibitory concentrations that gave a 50% increase in fluorescence intensity was determined (IC₅₀). The cKi values were determined from the IC₅₀ values according to Cer et al, Nucleic Acids Res, 2009, Jul 1;37(WebServer issue): W441-W445.

| | Bcl-2 wild-type K_{I} mean (M) | Bcl-2 Gly101Val mutant K_{I} mean (M) | Bcl-2 Asp103Tyr mutant K_{I} mean (M) | Bcl-2 Asp 103 Val mutant K_{I} mean (M) | Bcl-2 Asp103Glu mutant K_{I} mean (M) | Bcl-2 Arg129Leu mutant K_{I} mean (M) | Bcl-2 Ala 113 Gly Mutant K_{I} mean (M) |
|---|---|---|---|---|---|---|---|
| ABT-199 | 1.2E-09 | 9.8E-08 | inactive @1 µM | 5.9E-08 | 1.7E-08 | 1.2E-08 | 4.1E-09 |
| Compound A | 1.3E-09 | 2.3E-08 | 5.6E-09 | 3.7E-09 | 2.3E-09 | 3.3E-09 | 7.5E-09 |
| Compound B | 1.3E-10 | 1.9E-09 | 5.3E-10 | 2.8E-10 | 2.8E-10 | 1.0E-09 | 9.6E-10 |

### For Compound A and Compound B, the data are inhibition constants (K_{I}) determined from complete binding inhibition curves (cK_{I}), whilst the data for ABT-199 are estimated from incomplete binding inhibition curves (eKi's) in most cases due to low activity (c: complete; e: estimated)

| | Bcl-2 wild-type K_{I} mean (M) | Ratio Gly101Val /Wild-type | Ratio Asp103Tyr /Wild-type | Ratio Asp103Val /Wild-type | Ratio Asp 103 Glu /Wild-type | Ratio Arg129Leu /Wild-type | Ratio Ala 113 Gly /Wild-type |
|---|---|---|---|---|---|---|---|
| ABT-199 | 1.2E-09 | 84 | *N.C* | 50 | 14 | 11 | 4 |
| Compound A | 1.3E-09 | 18 | 4 | 3 | 2 | 3 | 6 |
| Compound B | 1.3E-10 | 15 | 4 | 2 | 2 | 8 | 8 |

| | | | | | | | |
|---|---|---|---|---|---|---|---|
| *N.C:* not calculated | | | | | | | |

The data demonstrate a significant loss of activity of ABT-199 on the Bcl-2 Asp103Tyr, Bcl-2 Asp103Val and Bcl-2 Asp103Glu mutants as compared to the Bcl-2 wild-type protein, whilst the affinities of Compound A and Compound B are slightly affected by the mutations. The affinity of Compound B is moderately affected by the Arg129Leu and the Ala113Gly mutations. Despite this, Compound B is 12-fold and 4-fold more potent than ABT-199 on the Arg129Leu and the Ala113Gly mutants, respectively.

### EXAMPLE 5: In vitro cytotoxicity of Compound B and ABT-199 in modified cells expressing Asp103Tyr mutant

### Material and methods

Cell lines were grown at 37 °C in a humidified atmosphere with 5% CO₂ in media recommended by the suppliers. KMS-12-PE (ACC 606) from the Leibniz-Institute DSMZ (Braunschweig, Germany). Lentiviral particles containing Bcl-2 mutated on D103Y were cloned into pcLV-CMV-DEST-IRES-TagRFP. Lentiviral particles (1 × 10⁶) were mixed with Polybrene at 8 µg/ml and transduced by spinoculation for 1h at 32 °C and incubated overnight. TagRFP positive- KMS-12-PE cells were sorted by FACS after 8 days. Bcl-2 expression was monitored by immunoblotting using anti-Flag and anti-Bcl-2 antibodies. Cells were seeded into 96-well plates and treated at 9 points with 1:3.16 serial dilution of compounds. Cell viability was assessed using CellTiter-Glo^{®} reagent following treatment with ABT-199 and Compound B for 72h. Results were normalized to the viability of cells without compounds (control wells). The C₅₀ values were calculated using nonlinear regression algorithms in XCell software.

### Results

| **Compounds** | **KMS-12-PE Bcl-2 D103Y (C50; nM)** |
|---|---|
| Compound B | 83.7 |
| ABT-199 | 1830 |

### Conclusion

These results demonstrated a shift of potency between ABT-199 and Compound B in cellular assays using KMS-12-PE cell lines overexpressing BCL2 D103Y. Compound B showed about 22-fold higher potency than ABT-199 in cell lines overexpressing the Bcl-2 D103Y mutant.

Cell lines overexpressing other Bcl-2 clinical mutants including D103V, D103E, R129L and A113G mutants are currently being set up using same the same protocol to evaluate the compounds mentioned above.

Further *in vivo* experiments based on xenograft Models derived from modified cells expressing the Bcl-2 mutants (including the modified cell lines described in Examples 2 and 5) may show that Compound B could be an effective therapy for treating Bcl-2 mediated cancer carrying Bcl-2 mutations such as G101V and/or D103Y and/or others. Additional results may be obtained by testing the efficacy of Compound B in ex vivo samples from CLL patients carrying at least one of the Bcl-2 mutations selected from G101V, D103Y, D103V, D103E, R129L and A113G using a cell viability assay.

## Claims

1. A Bcl-2 inhibitor for use in the treatment of Bcl-2 mediated cancer carrying at least 1, 2, 3, 4, 5 or all of the following mutations: (i) Gly101 Val; (ii) Asp103Tyr; (iii) Asp103Val; (iv) Asp103Glu; (v) Arg129Leu and (vi) Ala113Gly;
wherein the Bcl-2 inhibitor is selected from the group consisting of *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide (Compound A) and 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide (Compound B), or a pharmaceutically acceptable salt thereof.

2. A Bcl-2 inhibitor for use in the treatment of Bcl-2 mediated cancer according to claim 1 wherein the cancer carries the Gly101Val mutation.

3. A Bcl-2 inhibitor for use in the treatment of Bcl-2 mediated cancer according to claim 1 wherein the cancer carries the Asp103Tyr mutation.

4. A Bcl-2 inhibitor for use in the treatment of Bcl-2 mediated cancer according to claim 1 wherein the cancer carries the Asp103Val mutation.

5. A Bcl-2 inhibitor for use in the treatment of Bcl-2 mediated cancer according to claim 1 wherein the cancer carries the Asp103Glu mutation.

6. A Bcl-2 inhibitor for use according to any of claims 1 to 5, wherein the Bcl-2 mediated cancer is chronic lymphocytic leukemia (CLL).

7. A Bcl-2 inhibitor for use according to any of claims 1 to 5, wherein the Compound A is in the form of a hydrochloride salt.

8. A Bcl-2 inhibitor for use according to any of claims 1 to 5, wherein the Compound B is in the form of a hydrogen sulfate salt.

9. A Bcl-2 inhibitor for use according to any of claims 1 to 5, wherein the Compound B is in the form of a hydrochloride salt.

10. A Bcl-2 inhibitor for use according to any of claims 1 to 5, wherein Compound B is administered intravenously.

11. A pharmaceutical composition comprising a Bcl-2 inhibitor according to any of claims 1 to 10 for use in the treatment of Bcl-2 mediated cancer carrying at least 1, 2, 3, 4, 5 or all of the following mutations: (i) Gly101Val; (ii) Asp103Tyr; (iii) Asp103Val; (iv) Asp103Glu; (v) Arg129Leu and (vi) Ala113Gly.

12. A pharmaceutical composition comprising a Bcl-2 inhibitor according to claim 11 for use in the treatment of Bcl-2 mediated cancer carrying the Gly101Val mutation.

13. A pharmaceutical composition comprising a Bcl-2 inhibitor according to claim 11 for use in the treatment of Bcl-2 mediated cancer carrying the Asp103Tyr mutation.

14. A pharmaceutical composition comprising a Bcl-2 inhibitor according to claim 11 for use in the treatment of Bcl-2 mediated cancer carrying the Asp 103 Val mutation.

15. A pharmaceutical composition comprising a Bcl-2 inhibitor according to claim 11 for use in the treatment of Bcl-2 mediated cancer carrying the Asp103Glu mutation.

16. A therapeutically effective amount of a Bcl-2 inhibitor according to any of claims 1 to 10 for use in a method of sensitizing a patient having a Bcl-2 mediated cancer carrying at least 1, 2, 3, 4, 5 or all of the following mutations: (i) Gly101 Val; (ii) Asp103Tyr; (iii) Asp103Val; (iv) Asp103Glu; (v) Arg129Leu and (vi) Ala113Gly;
who is (a) refractory to at least one anti-cancer agent, or (b) in relapse after treatment with an anti-cancer agent, or both (a) and (b).

17. A therapeutically effective amount of a Bcl-2 inhibitor according to any of claims 1 to 10 for use in a method of sensitizing a patient having a Bcl-2 mediated cancer carrying the Gly101Val mutation who is (i) refractory to at least one anti-cancer agent, or (ii) in relapse after treatment with an anti-cancer agent, or both (i) and (ii).

18. A therapeutically effective amount of a Bcl-2 inhibitor for use according to any of claims 16 or 17, wherein the anti-cancer agent is a targeted therapy.

19. A therapeutically effective amount of a Bcl-2 inhibitor for use according to claim 18 wherein the cancer is chronic lymphocytic leukemia (CLL).

20. A therapeutically effective amount of a Bcl-2 inhibitor for use according to any of claims 18 or 19 wherein the targeted therapy is venetoclax (ABT-199).

21. A Bcl-2 inhibitor for use in a method of treating a Bcl-2 mediated cancer in a patient, comprising the steps of:
(a) obtaining a biological sample from said patient and detecting whether the biological sample comprises at least 1, 2, 3, 4, 5 or all of the following mutations: (i) Gly101Val; (ii) Asp103Tyr; (iii) Asp103Val;(iv) Asp103Glu; (v) Arg129Leu and (vi) Ala113Gly;
(b) identifying said patients as having reduced likelihood of response to venetoclax;
(c) administering a therapeutically effective amount of a Bcl-2 inhibitor according to any of claims 1 to 10, to said patient based on the presence of the thus detected mutations.

22. A Bcl-2 inhibitor for use in a method of treating a Bcl-2 mediated cancer in a patient, comprising the steps of:
(a) obtaining a biological sample from said patient and detecting whether the biological sample comprises the Gly101Val mutation;
(b) identifying said patients as having reduced likelihood of response to venetoclax;
(c) administering a therapeutically effective amount of a Bcl-2 inhibitor according to any of claims 1 to 10, to said patient based on the presence of the Gly101Val mutation.

## Patentansprüche

1. Bcl-2-Inhibitor zur Verwendung bei der Behandlung von Bc1-2-vermitteltem Krebs, der mindestens 1, 2, 3, 4, 5 oder alle der folgenden Mutationen trägt: (i) Gly101Val; (ii) Asp103Tyr; (iii) Asp103Val;(iv) Asp103Glu; (v) Arg129Leu und (vi) A1a113Gly;
wobei der Bc1-2-Inhibitor ausgewählt ist aus der Gruppe bestehend aus *N*-(4-Hydroxyphenyl)-3-{6-[((3*S*)-3-(4-morpholinylmethyl)-3,4-dihydro-2(1*H*)-isochinolinyl)carbonyl]-1,3-benzodioxol-5-yl}-*N*-phenyl-5,6,7,8-tetrahydro-1-indolizincarboxamid (Verbindung A) und 5-(5-Chlor-2-{[(3*S*)-3-(morpholin-4-ylmethyl)-3,4-dihydroisochinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrol-3-carboxamid (Verbindung B) oder einem pharmazeutisch verträglichen Salz davon.

2. Bc1-2-Inhibitor zur Verwendung bei der Behandlung von Bc1-2-vermitteltem Krebs nach Anspruch 1, wobei der Krebs die Gly101Val-Mutation trägt.

3. Bc1-2-Inhibitor zur Verwendung bei der Behandlung von Bc1-2-vermitteltem Krebs nach Anspruch 1, wobei der Krebs die Asp103Tyr-Mutation trägt.

4. Bc1-2-Inhibitor zur Verwendung bei der Behandlung von Bc1-2-vermitteltem Krebs nach Anspruch 1, wobei der Krebs die Asp103Val-Mutation trägt.

5. Bc1-2-Inhibitor zur Verwendung bei der Behandlung von Bc1-2-vermitteltem Krebs nach Anspruch 1, wobei der Krebs die Asp103Glu-Mutation trägt.

6. Bc1-2-Inhibitor zur Verwendung nach einem der Ansprüche 1 bis 5, wobei es sich bei dem Bc1-2 vermittelten Krebs um chronische lymphatische Leukämie (CLL) handelt.

7. Bc1-2-Inhibitor zur Verwendung nach einem der Ansprüche 1 bis 5, wobei die Verbindung A in Form eines Hydrochloridsalzes vorliegt.

8. Bc1-2-Inhibitor zur Verwendung nach einem der Ansprüche 1 bis 5, wobei die Verbindung B in Form eines Hydrogensulfatsalzes vorliegt.

9. Bc1-2-Inhibitor zur Verwendung nach einem der Ansprüche 1 bis 5, wobei die Verbindung B in Form eines Hydrochloridsalzes vorliegt.

10. Bc1-2-Inhibitor zur Verwendung nach einem der Ansprüche 1 bis 5, wobei Verbindung B intravenös verabreicht wird.

11. Pharmazeutische Zusammensetzung, umfassend einen Bc1-2-Inhibitor nach einem der Ansprüche 1 bis 10, zur Verwendung bei der Behandlung von Bc1-2-vermitteltem Krebs, der mindestens 1, 2, 3, 4, 5 oder alle der folgenden Mutationen trägt: (i) Gly101Val; (ii) Asp103Tyr; (iii) Asp103Val;(iv) Asp103Glu; (v) Arg129Leu und (vi) A1a113Gly.

12. Pharmazeutische Zusammensetzung, umfassend einen Bc1-2-Inhibitor nach Anspruch 11 zur Verwendung bei der Behandlung von Bc1-2-vermitteltem Krebs, der die Gly101Val-Mutation trägt.

13. Pharmazeutische Zusammensetzung, umfassend einen Bc1-2-Inhibitor nach Anspruch 11 zur Verwendung bei der Behandlung von Bc1-2-vermitteltem Krebs, der die Asp103Tyr-Mutation trägt.

14. Pharmazeutische Zusammensetzung, umfassend einen Bc1-2-Inhibitor nach Anspruch 11 zur Verwendung bei der Behandlung von Bc1-2-vermitteltem Krebs, der die Asp103Val-Mutation trägt.

15. Pharmazeutische Zusammensetzung, umfassend einen Bc1-2-Inhibitor nach Anspruch 11 zur Verwendung bei der Behandlung von Bc1-2-vermitteltem Krebs, der die Asp103Glu-Mutation trägt.

16. Therapeutisch wirksame Menge eines Bc1-2-Inhibitors nach einem der Ansprüche 1 bis 10 zur Verwendung in einem Verfahren zur Sensibilisierung eines Patienten mit einem Bc1-2-vermittelten Krebs, der mindestens 1, 2, 3, 4, 5 oder alle der folgenden Mutationen trägt:
(i) Gly101Val; (ii) Asp103Tyr; (iii) Asp103Val;(iv) Asp103Glu; (v) Arg129Leu und (vi) A1a113Gly;
der (a) refraktär gegenüber mindestens einem Antikrebsmittel ist oder (b) nach der Behandlung mit einem Antikrebsmittel einen Rückfall erlitten hat, oder beides (a) und (b).

17. Therapeutisch wirksame Menge eines Bc1-2-Inhibitors nach einem der Ansprüche 1 bis 10 zur Verwendung in einem Verfahren zur Sensibilisierung eines Patienten mit einem Bc1-2-vermittelten Krebs, der die Gly101Val-Mutation trägt, der (i) refraktär gegenüber mindestens einem Antikrebsmittel ist oder (ii) nach der Behandlung mit einem Antikrebsmittel einen Rückfall erlitten hat, oder beides (i) und (ii).

18. Therapeutisch wirksame Menge eines Bc1-2-Inhibitors zur Verwendung nach einem der Ansprüche 16 oder 17, wobei das Antikrebsmittel eine gezielte Therapie ist.

19. Therapeutisch wirksame Menge eines Bc1-2-Inhibitors zur Verwendung nach Anspruch 18, wobei es sich bei dem Krebs um chronische lymphatische Leukämie (CLL) handelt.

20. Therapeutisch wirksame Menge eines Bc1-2-Inhibitors zur Verwendung nach einem der Ansprüche 18 oder 19, wobei es sich bei der gezielten Therapie um Venetoclax (ABT-199) handelt.

21. Bc1-2-Inhibitor zur Verwendung in einem Verfahren zur Behandlung eines Bc1-2-vermittelten Krebses bei einem Patienten, umfassend die Schritte:
(a) Gewinnen einer biologischen Probe von dem Patienten und Feststellen, ob die biologische Probe mindestens 1, 2, 3, 4, 5 oder alle der folgenden Mutationen umfasst: (i) Gly101Val; (ii) Asp103Tyr; (iii) Asp103Val;(iv) Asp103Glu; (v) Arg129Leu und (vi) A1a113Gly;
(b) Identifizieren dieser Patienten als solche, bei denen die Wahrscheinlichkeit einer Reaktion auf Venetoclax verringert ist;
(c) Verabreichen einer therapeutisch wirksamen Menge eines Bc1-2-Inhibitors nach einem der Ansprüche 1 bis 10 an den Patienten basierend auf dem Vorhandensein der so nachgewiesenen Mutationen.

22. Bc1-2-Inhibitor zur Verwendung in einem Verfahren zur Behandlung eines Bc1-2-vermittelten Krebses bei einem Patienten, umfassend die Schritte:
(a) Gewinnen einer biologischen Probe von dem Patienten und Feststellen, ob die biologische Probe die Gly101 Val-Mutation umfasst;
(b) Identifizieren dieser Patienten als solche, bei denen die Wahrscheinlichkeit einer Reaktion auf Venetoclax verringert ist;
(c) Verabreichen einer therapeutisch wirksamen Menge eines Bc1-2-Inhibitors nach einem der Ansprüche 1 bis 10 an den Patienten basierend auf dem Vorhandensein der Gly101Val-Mutation.

## Revendications

1. Inhibiteur de Bcl-2 destiné à être utilisé dans le traitement d'un cancer médié par Bcl-2 portant au moins 1, 2, 3, 4, 5 ou toutes les mutations suivantes : (i) Gly101Val ; (ii) Asp103Tyr ; (iii) Asp103Val ; (iv) Asp103Glu ; (v) Arg129Leu et (vi) Ala113Gly ;
dans lequel l'inhibiteur de Bcl-2 est choisi dans le groupe constitué par le N-(4-hydroxyphényl)-3-{6-[((3S)-3-(4-morpholinylméthyl)-3,4-dihydro-2(1*H*)-isoquinolinyl)carbonyl]-1,3-benzodioxol-5-yl}-*N*-phényl-5,6,7,8-tétrahydro-1-indolizine carboxamide (composé A) et le 5-(5-chloro-2-{[(3S)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N-*(4-hydroxyphényl)-1,2-diméthyl-1*H*-pyrrole-3-carboxamide (composé B), ou un sel pharmaceutiquement acceptable de ceuxci .

2. Inhibiteur de Bcl-2 destiné à être utilisé dans le traitement d'un cancer médié par Bcl-2 selon la revendication 1, dans lequel le cancer porte la mutation Gly101Val.

3. Inhibiteur de Bcl-2 destiné à être utilisé dans le traitement d'un cancer médié par Bcl-2 selon la revendication 1, dans lequel le cancer porte la mutation Asp103Tyr.

4. Inhibiteur de Bcl-2 destiné à être utilisé dans le traitement d'un cancer médié par Bcl-2 selon la revendication 1, dans lequel le cancer porte la mutation Asp103Val.

5. Inhibiteur de Bcl-2 destiné à être utilisé dans le traitement d'un cancer médié par Bcl-2 selon la revendication 1, dans lequel le cancer porte la mutation Asp103Glu.

6. Inhibiteur de Bcl-2 destiné à être utilisé selon l'une quelconque des revendications 1 à 5, dans lequel le cancer médié par Bcl-2 est la leucémie lymphoïde chronique (CLL).

7. Inhibiteur de Bcl-2 destiné à être utilisé selon l'une quelconque des revendications 1 à 5, dans lequel le composé A est sous la forme d'un sel chlorhydrate.

8. Inhibiteur de Bcl-2 destiné à être utilisé selon l'une quelconque des revendications 1 à 5, dans lequel le composé B est sous la forme d'un sel hydrogénosulfate.

9. Inhibiteur de Bcl-2 destiné à être utilisé selon l'une quelconque des revendications 1 à 5, dans lequel le composé B est sous la forme d'un sel chlorhydrate.

10. Inhibiteur de Bcl-2 destiné à être utilisé selon l'une quelconque des revendications 1 à 5, dans lequel le composé B est administré par voie intraveineuse.

11. Composition pharmaceutique comprenant un inhibiteur de Bcl-2 selon l'une quelconque des revendications 1 à 10 destinée à être utilisée dans le traitement d'un cancer médié par Bcl-2 portant au moins 1, 2, 3, 4, 5 ou toutes les mutations suivantes : (i) Gly101Val ; (ii) Asp103Tyr ; (iii) Asp103Val ; (iv) Asp103Glu ; (v) Arg129Leu et (vi) Ala113Gly.

12. Composition pharmaceutique comprenant un inhibiteur de Bcl-2 selon la revendication 11 destinée à être utilisée dans le traitement d'un cancer médié par Bcl-2 portant la mutation Gly101Val.

13. Composition pharmaceutique comprenant un inhibiteur de Bcl-2 selon la revendication 11 destinée à être utilisée dans le traitement d'un cancer médié par Bcl-2 portant la mutation Asp103Tyr.

14. Composition pharmaceutique comprenant un inhibiteur de Bcl-2 selon la revendication 11 destinée à être utilisée dans le traitement d'un cancer médié par Bcl-2 portant la mutation Asp103Val.

15. Composition pharmaceutique comprenant un inhibiteur de Bcl-2 selon la revendication 11 destinée à être utilisée dans le traitement d'un cancer médié par Bcl-2 portant la mutation Asp103Glu.

16. Quantité thérapeutiquement efficace d'un inhibiteur de Bcl-2 selon l'une quelconque des revendications 1 à 10 destinée à être utilisée dans un procédé de sensibilisation d'un patient ayant un cancer médié par Bcl-2 portant au moins 1, 2, 3, 4, 5 ou toutes les mutations suivantes : (i) Gly101Val ; (ii) Asp103Tyr ; (iii) Asp103Val ; (iv) Asp103Glu ; (v) Arg129Leu et (vi) Ala113Gly ;
qui est (a) réfractaire à au moins un agent anticancéreux, ou (b) en rechute après un traitement avec un agent anticancéreux, ou à la fois (a) et (b).

17. Quantité thérapeutiquement efficace d'un inhibiteur de Bcl-2 selon l'une quelconque des revendications 1 à 10 destinée à être utilisée dans un procédé de sensibilisation d'un patient ayant un cancer médié par Bcl-2 portant la mutation Gly101Val qui est (i) réfractaire à au moins un agent anticancéreux, ou (ii) en rechute après un traitement avec un agent anticancéreux, ou à la fois (i) et (ii).

18. Quantité thérapeutiquement efficace d'un inhibiteur de Bcl-2 destinée à être utilisée selon l'une quelconque des revendications 16 ou 17, dans laquelle l'agent anticancéreux est une thérapie ciblée.

19. Quantité thérapeutiquement efficace d'un inhibiteur de Bcl-2 destinée à être utilisée selon la revendication 18, dans laquelle le cancer est la leucémie lymphoïde chronique (CLL).

20. Quantité thérapeutiquement efficace d'un inhibiteur de Bcl-2 destinée à être utilisée selon l'une quelconque des revendications 18 ou 19, dans laquelle la thérapie ciblée est le vénétoclax (ABT-199).

21. Inhibiteur de Bcl-2 destiné à être utilisé dans un procédé de traitement d'un cancer médié par Bcl-2 chez un patient, comprenant les étapes suivantes :
(a) obtention d'un échantillon biologique auprès dudit patient et détection pour déterminer si l'échantillon biologique comprend au moins 1, 2, 3, 4, 5 ou toutes les mutations suivantes : (i) Gly101Val ; (ii) Asp103Tyr ; (iii) Asp103Val ; (iv) Asp103Glu ; (v) Arg129Leu et (vi) Ala113Gly ;
(b) identification desdits patients comme ayant une probabilité réduite de réponse au vénétoclax ;
(c) administration d'une quantité thérapeutiquement efficace d'un inhibiteur de Bcl-2 selon l'une quelconque des revendications 1 à 10, audit patient sur la base de la présence des mutations ainsi détectées.

22. Inhibiteur de Bcl-2 destiné à être utilisé dans un procédé de traitement d'un cancer médié par Bcl-2 chez un patient, comprenant les étapes suivantes :
(a) obtention d'un échantillon biologique auprès dudit patient et détection pour déterminer si l'échantillon biologique comprend la mutation Gly101Val ;
(b) identification desdits patients comme ayant une probabilité réduite de réponse au vénétoclax ;
(c) administration d'une quantité thérapeutiquement efficace d'un inhibiteur de Bcl-2 selon l'une quelconque des revendications 1 à 10, audit patient sur la base de la présence de la mutation Gly101Val.
